# EUROPEAN PATENT APPLICATION

(11) **EP 4 122 502 A1**
(43) Date of publication of application: **25.01.2023**
(21) Application number: 22185633.9
(22) Date of filing: 19.07.2022
(51) Int. Cl.: A61L 2/10, A45C 15/06, F21V 9/00, G02B 6/00, G02F 1/37, F21V 8/00

(54) **DIRECTIONAL UVC SYSTEMS AND METHODS**

(30) Priority: 23.07.2021 US 202117383796
(71) Applicant: Goodrich Corporation, Charlotte, NC 28217-4578 (US)
(72) Inventor: GULER, Urcan, East Hartford, CT, 06118 (US); MANTESE, Joseph, Ellington, 06029 (US); PEARSON, Matthew, Hartford, 06106 (US)
(74) Representative: Dehns

(57) **Abstract**

A portable sanitization system (200) may comprise: a portable device (210); a light source disposed in the portable device (210), the light source configured to emit a light having a wavelength between 414 and 474 nm; a light converter (230) comprising a nonlinear crystal configured for frequency doubling; and a light guide (220) extending from the light source to the light converter (230), the light converter (230) disposed proximal a tip of the light guide.

## Description

### FIELD

The present disclosure relates generally to directional UV-C light systems and methods, more particularly, to portable UV-C light systems and methods.

### BACKGROUND

Ultraviolet radiation Type C, having a wavelength of between 200-280 nm (UVC) is effective for surface treatment of bacterial and influenza pathogens. 254 nm UVC generation is possible via LEDs but can be a health hazard to human skin and eyes. 222 nm is particularly favorable as it is commonly considered safe for humans and could be used for disinfecting surfaces without the use of chemicals. Unfortunately, UVC lasers and LEDs are not readily available, limiting UVC generation devices generally to excimer (gas-discharge) bulbs that have a short-life, produce excessive heat when in use, and are non-directional.

### SUMMARY

A portable sanitization system is disclosed herein. The portable sanitization system may comprise: a portable device; a light source disposed in the portable device, the light source configured to emit a light having a wavelength between 414 and 474 nm; a light converter comprising a nonlinear crystal configured for frequency doubling; and a light guide extending from the light source to the light converter, the light converter disposed proximal a tip of the light guide.

In various embodiments, the light guide comprises a fiber optic cable. The light source may comprise a laser pump source. The portable sanitization system may further comprise a beam directing lens configured to direct converted light from the light converter. The light guide may comprise a flexible portion and a rigid portion. The portable sanitization system may further comprise a trigger coupled to the rigid portion. The trigger may be in operable communication with the light source. The light source may be configured to generate the light having the wavelength between 414 and 474 nm in response to the trigger being actuated. The light converter may be configured to convert a portion of the light from the wavelength between 414 and 474 nm to a second wavelength between 207 nm and 237 nm.

A sanitization device is disclosed herein. The sanitization device may comprise: a light guide; a light source configured to emit a light having a wavelength between 414 and 474 nm, the light source being portable; and a light converter having a nonlinear crystal, the light guide extending from the light source to the light converter, the light converter configured to convert at least a portion of the wavelength to a second wavelength between 207 nm and 247 nm.

In various embodiments, the sanitization device may further comprise a backpack, the light guide disposed in the backpack. The light guide may comprise a fiber optic cable and a rod, the rod configured to house the light converter. The sanitization device may further comprise a beam directing lens disposed proximal a tip of the light guide. The light converter may be disposed proximal the tip of the light guide. The light source may comprise one of a light emitting diode (LED), a Nd:YAG/LBO laser, a InGaN laser diode, an InGaN laser pump source. The light guide may comprise a flexible portion and a rigid portion. The flexible portion may extend from the light source to the rigid portion, and the rigid portion may extend from the flexible portion to a tip of the light guide.

A method of using a portable sanitization system is disclosed herein. The method may comprise: generating a light having a first wavelength between 414 and 474 nm; guiding the light from a first location to a second location; converting at least a portion of the light from the first wavelength to a second wavelength between 207 and 247 nm; directing the light having the second wavelength toward a surface; and in response to directing the light toward the surface, the surface is at least partially sanitized.

In various embodiments, the first location is disposed in a portable device, and the second location is disposed proximal a tip of light guide extending from the portable device. The method may further comprise activating a trigger prior to generating the light having the first wavelength.

The forgoing features and elements may be combined in various combinations without exclusivity, unless expressly indicated herein otherwise. These features and elements as well as the operation of the disclosed embodiments will become more apparent in light of the following description and accompanying drawings.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the following detailed description and claims in connection with the following drawings. While the drawings illustrate various embodiments employing the principles described herein, the drawings do not limit the scope of the claims.
FIG. 1 illustrates a sanitization process in accordance with various embodiments;
FIG. 2A illustrates a directional UVC system and device, in accordance with various embodiments;
FIG. 2B illustrates second harmonic generation, in accordance with various embodiments;
FIG. 2C illustrates a schematic view of a light converter, in accordance with various embodiments;
FIG. 2D illustrates a nonlinear crystal configured for frequency doubling, in accordance with various embodiments; and
FIG. 3 illustrates a schematic view of a control system for a direction UVC system and device, in accordance with various embodiments.

### DETAILED DESCRIPTION

The following detailed description of various embodiments herein makes reference to the accompanying drawings, which show various embodiments by way of illustration. While these various embodiments are described in sufficient detail to enable those skilled in the art to practice the disclosure, it should be understood that other embodiments may be realized and that changes may be made without departing from the scope of the disclosure. Thus, the detailed description herein is presented for purposes of illustration only and not of limitation. Furthermore, any reference to singular includes plural embodiments, and any reference to more than one component or step may include a singular embodiment or step. Also, any reference to attached, fixed, connected, or the like may include permanent, removable, temporary, partial, full or any other possible attachment option. Additionally, any reference to without contact (or similar phrases) may also include reduced contact or minimal contact. It should also be understood that unless specifically stated otherwise, references to "a," "an" or "the" may include one or more than one and that reference to an item in the singular may also include the item in the plural. Further, all ranges may include upper and lower values and all ranges and ratio limits disclosed herein may be combined.

In various embodiments, ultraviolet radiation (UVC) (e.g., between 200 and 280 nm wavelength light) has a high intensity, high power discharge lamps. Typical UVC generating sources are very large, heavy, and not portable. Additionally, typical UVC generating sources cannot direct the light as the light is being generated proximal the source and does not reflect or bend in a manner to allow directing the light.

In various embodiments, systems and methods disclosed herein utilize a ready means of providing UVC devices through non-linear optical device for frequency doubling; thereby permitting the use of commercially available 444nm blue lasers to provide approximately 222nm UVC germicidal light for pathogen surface decontamination. In various embodiments, the systems and methods store the laser source in a backpack or remote device and guides pump light through a fiber optic cable to a nonlinear optical assembly attached to the fiber end, allowing directional UVC exposure analogous to a water hose. Beam shape (e.g., line, square) can be changed with plug-in optics and the nonlinear assembly at the fiber tip is also replaceable.

In various embodiments, optical flood decontamination minimizes variability, provides rapid decontamination, and enables future automation. Approximately 222 nm is particularly advantageous due to effectiveness and safety. In various embodiments, the systems and methods disclosed herein facilitate all the advantages of 222 nm UVC decontamination with additional benefits such as directional exposure and compact, modular design for easy operation and maintenance. The systems and methods allows the light source and lens to be separately located for easy beam-steering/scanning either automated or manual.

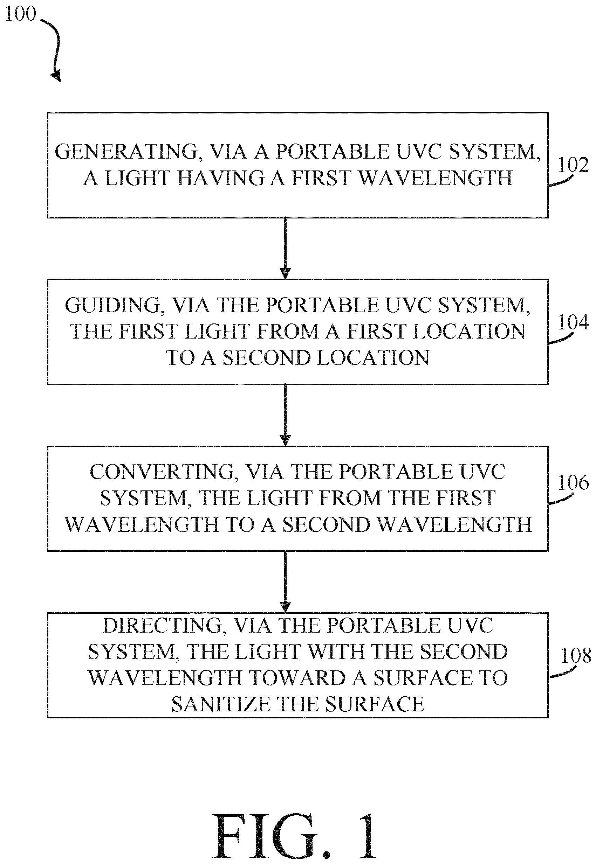

Referring now to FIG. 1, a method of using a portable UVC system, method 100, is illustrated in accordance with various embodiments. The method 100 comprises generating, via a portable UVC system, a light having a first wavelength (step 102). In various embodiments, the first wavelength is between 414 nm and 474 nm, or between 429 nm and 459 nm, or approximately 444 nm +/- 10 nm. In various embodiments, the portable UVC system comprises a light source. In various embodiments, the light source may comprise a light emitting diode (LED), a Nd:YAG/LBO laser, a InGaN laser diode, an InGaN laser pump source or the like. In various embodiments, any light source capable of generating a light with a first wavelength between 414 nm and 474 nm is within the scope of this disclosure. In various embodiments, the light source may weigh significantly less than a light source capable of generating a UVC wavelength (e.g., between 200 nm and 280 nm).

In various embodiments, the method 100 further comprises guiding, via the portable UVC system, the first light from a first location to a second location (step 104). In various embodiments, the portable UVC system comprises a guiding component (e.g., a fiber optic cable or the like) configured to guide the first light from the first location to the second location. In this regard, the portable UVC system is configured to allow the light having the first wavelength from the light source to an output location in accordance with various embodiments. In various embodiments, the output location may be immediately adjacent to the light source. The output location may include a tip or the like configured to guide a converted light to a respective surface as described further herein.

In various embodiments, the method 100 further comprises converting, via the portable UVC system, the light from the first wavelength to a second wavelength (step 106). In various embodiments, the conversion is performed proximate the output of the portable UVC system. For example, the portable UVC system may comprises a light conversion device proximate the tip. In this regard, the light conversion device may be configured to convert the light from the first wavelength to the second wavelength prior to output the light. In various embodiments, the light conversion device is configured to halve the wavelength of the light. In this regard, the second wavelength may be between 207 nm and 237 nm, or between 215 nm and 230 nm, or approximately 222 nm, in accordance with various embodiments.

In various embodiments, the method 100 further comprises directing, via the portable UVC system, the light with the second wavelength toward a surface to sanitize the surface (step 108). In this regard, the UVC system may comprise a grip capable of being held by a user proximate the tip for the user to guide the converted light from step 106 towards a surface to sanitize, in accordance with various embodiments.

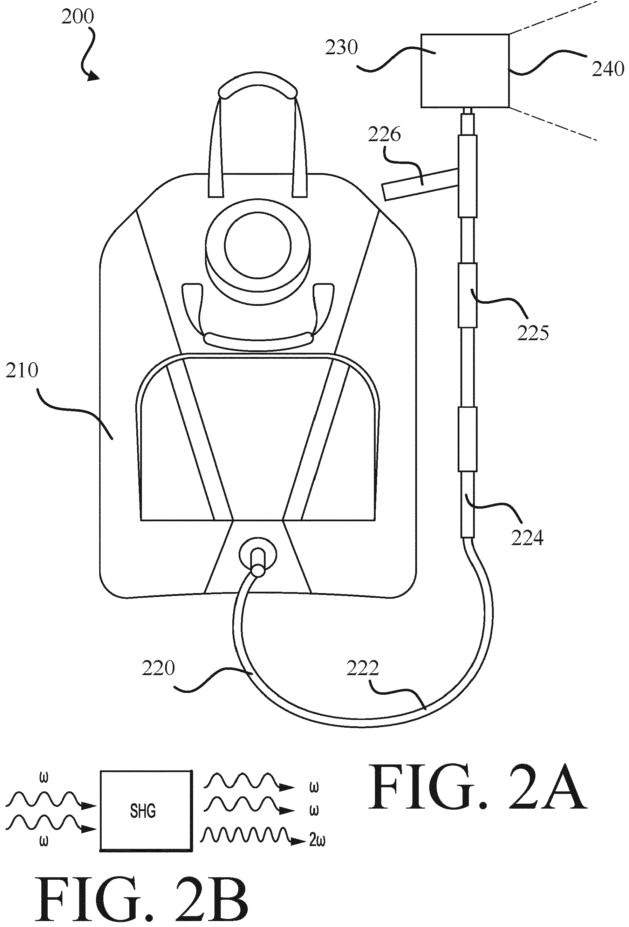

Referring now to FIG. 2A, a front view of a portable UVC light system 200 is illustrated, in accordance with various embodiments. The portable UVC light system comprises a portable device 210, a light guide 220, and a light converter 230, and a light output 240. In various embodiments, the portable device 210 may comprise a backpack, a case, a bag, or the like. In various embodiments, the portable device 210 is any device capable of carrying a light source as described previously herein.

In various embodiments, the light guide 220 may comprises a liquid light guide, a fiber optic cable, or the like. In various embodiments, the light guide 220 comprises a fiber optic cable. In various embodiments, the light guide 220 is configured to guide a beam having the first wavelength from step 102 of method 100 as defined above. In this regard, guiding approximately 444 nm wavelength light in a medium is significantly easier relative to guiding a wavelength of approximately 222 nm wavelength light. Thus, guiding the beam at 444 nm via the light guide 220, in accordance with step 102 of method 100 from FIG. 1, and converting to 222 nm just before exit (e.g., via light converter 230), in accordance with step 106 of method 100 from FIG. 1, has a significant practical advantage, in accordance with various embodiments as described further herein.

In various embodiments, the light guide 220 may comprise a cable 222, a rod 224 and a trigger 226. In various embodiments, the cable 222 may be flexible in order to allow relatively easy movement of the cable. In various embodiments, the rod 224 may be a rigid component. In this regard, the rod 224 may be configured to be held by a user sanitizing an aircraft cabin, or any other area to be sanitized, in accordance with various embodiments. The rod 224 may comprise a grip 225, in accordance with various embodiments. The grip 225 may provide an ergonomic advantage to a user, in accordance with various embodiments. In various embodiments, the trigger 226 may be in operable (i.e., electrical or mechanical) communication with the light source disposed within the portable device 210 as described further herein. In this regard, in response to actuating the trigger 226, the light source disposed within the portable device 210 may activate and trigger the method 100 from FIG. 1.

In various embodiments, the light converter 230 comprises a frequency doubling device. In this regard, the light converter 230 may contain a nonlinear crystal, which may be mounted in a crystal oven, in accordance with various embodiments. In various embodiments, the light converter 230 may further comprise an optical resonator and/or a feedback system for frequency locking. In various embodiments, the light converter 230 is configured for second-harmonic generation (SHG) as illustrated in FIG. 2B. Transparent crystalline crystal materials can exhibit different kinds of optical non-linearities which are associated with nonlinear polarization. In various embodiments, a crystal material which can be critically phase-matched at room temperature, because noncritical phase matching often involves the operation of the crystal in a temperature-stabilized crystal oven.

In various embodiments, the light converter 230 at the tip of the light guide 220, may be configured as a removeable component (i.e., a plug in replaceable part). In this regard, by having the light converter 230 be a 'replaceable' part, the portable UVC light system 200 from FIG. 2A may provide a practical advantage over other approaches where there would be several difficulties due to component access in accordance with various embodiments. The high intensity pump beam would be focused and high energy UVC light would be generated proximal the tip only, which would potentially cause degradation in time and result in part replacement or maintenance that would be easier with this modular approach. Alignments (angles, relative positions, etc.) of the light converter 230 could be adjustable and customizable by using various light converter configurations. The rest of the system would be relatively stable and robust, in accordance with various embodiments.

In various embodiments, the light converter 230 comprises a nonlinear crystal, such as lithium niobate, lithium tantalate, LaBGeO5 (LBGO), potassium niobate, potassium titanyl phosphate, potassium dihydrogen phosphate, potassium dideuterium phosphate, lithium triborate, cesium lithium borate, β-barium borate, bismuth triborate, cesium borate, yttrium calcium oxyborate, strontium beryllium borate, zinc germanium diphosphide, silver gallium sulfide and selenide, gallium selenide, cadmium selenide, or the like. The present disclosure is not limited in this regard.

In various embodiments, the light output 240 may comprise a focusing barrel, a beam direction lens, or the like. In this regard, the light output 240 may be a converging lens, a diverging lens, or any other lens configured to direct an output beam from the UVC light system 200. In various embodiments, the UVC light system 200 may be configured to direct the output light beam to a surface to be sanitized in accordance with method 100 from FIG. 1. In this regard, the UVC light system 200 may include a direction beam facilitating targeting of areas to be sanitized as opposed to excimer lamps, which are non-directional, in accordance with various embodiments.

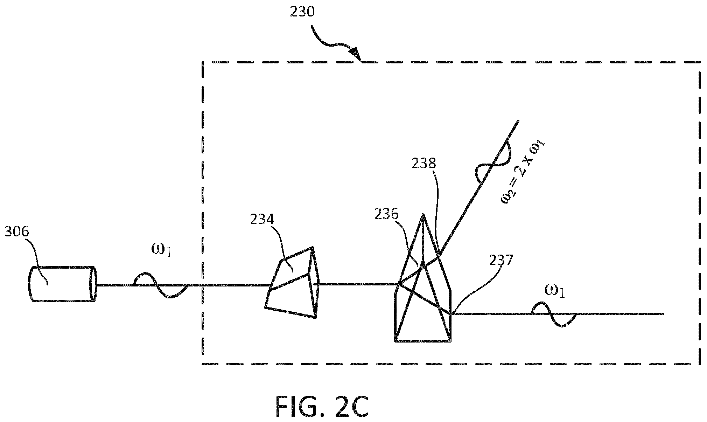

Referring now to FIG. 2C, a schematic view of the light converter from FIG. 2A is illustrated, in accordance with various embodiments. In various embodiments, the light converter 230 is in operable communication with a light source 306. In various embodiments, the light source 306 may comprise a light emitting diode (LED), a Nd:YAG/LBO laser, a InGaN laser diode, an InGaN laser pump source or the like. In various embodiments, any light source capable of generating a light with a first wavelength between 414 nm and 474 nm is within the scope of this disclosure. In various embodiments, the light source may weigh significantly less than a light source capable of generating a UVC wavelength (e.g., between 200 nm and 280 nm).

In various embodiments, the light converter 230 further comprises a nonlinear crystal 234. The nonlinear crystal 234 is configured to double a frequency of a portion of an incoming light (e.g., from the light source 306), in accordance with various embodiments. In various embodiments, the nonlinear crystal 234 is configured for second harmonic generation (SHG). Transparent crystalline crystal materials can exhibit different kinds of optical non-linearities which are associated with nonlinear polarization. In various embodiments, it can be highly desirable to use a crystal material which can be critically phase-matched at room temperature, because noncritical phase matching often involves the operation of the crystal in a temperature-stabilized crystal oven.

In various embodiments, the nonlinear crystal 204 may be any nonlinear crystal configured for frequency doubling, which may include lithium niobate, lithium tantalate, LaBGeO5 (LBGO), potassium niobate, potassium titanyl phosphate, potassium dihydrogen phosphate, potassium dideuterium phosphate, lithium triborate, cesium lithium borate, β-barium borate, bismuth triborate, cesium borate, yttrium calcium oxyborate, strontium beryllium borate, zinc germanium diphosphide, silver gallium sulfide and elenide, gallium selenide, cadmium selenide, or the like. The present disclosure is not limited in this regard.

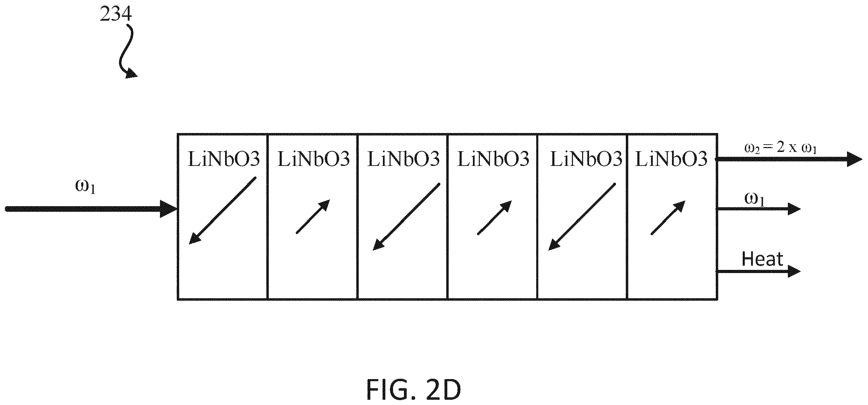

With brief reference to FIG. 2D, a nonlinear crystal 234 in accordance with various embodiments, is illustrated. The nonlinear crystal 234 may comprise alternating layers of a first semiconductor and a second semiconductor. For example, the first semiconductor may comprise a regular layer of lithium niobate (LiNbO₃) and the second semiconductor may comprise an inversion layer of LiNbO₃. The nonlinear crystal 234 is configured for second harmonic generation. In this regard, two photons of the same frequency interact with the nonlinear crystal 234, are combined, and generate a new photon with twice the energy of the initial photons. In various embodiments, as illustrated light having a first wavelength enters the nonlinear crystal 234 where nonlinear phase-matching stores energy within the nonlinear crystal and outputs a second harmonic wave (e.g., ω₂ = 2 × ω₁), a residual wave having the same wavelength as the input, and heat. In various embodiments, the second harmonic wave may have less energy relative to the input wave (e.g., between 0.1% and 10%, or between 3% and 10%). In this regard, the light converter 230 may have similar or better efficiency compared to UVC light sources, such as excimer lamps, which have approximately 3% efficiency. In various embodiments, even with efficiency below that of excimer lamps, the light converter 230 of the portable UVC light system 200 from FIG. 2A may still have additional benefits that outweigh the reduced efficiency, such as directionality, easier maintenance, reduced weight, greater mobility, and/or reduced safety concerns for a respective user. Thus, the systems and methods disclosed herein may result in a significantly lighter, portable, and/or less expensive UVC sanitization device, in accordance with various embodiments.

Referring back to FIG. 2C, In various embodiments, the nonlinear crystal 204 is configured to receive a light having a first wavelength and output a first portion of the light with the first wavelength, a second portion of the light with a second wavelength, the second wavelength double the first wavelength, and heat.

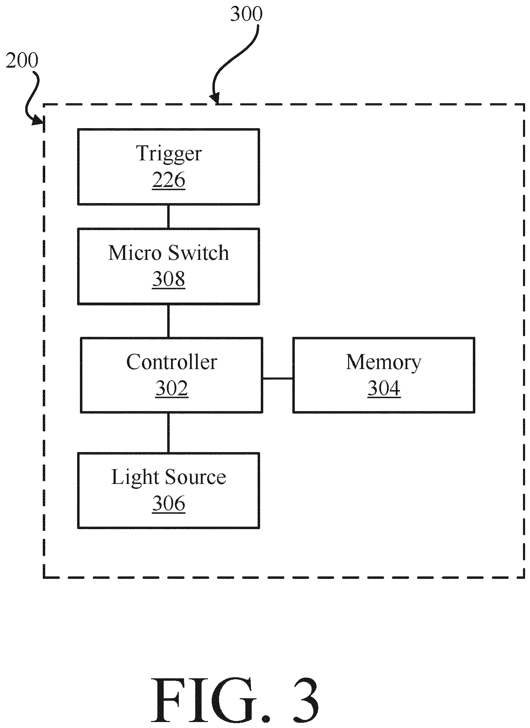

In various embodiments, the output from the nonlinear crystal 204 is received by a prism 206 configured to direct the light received from the nonlinear crystal. For example, the first portion of the light with the first wavelength may directed through first output 207 of the prism 206 and the second portion of the light with the second wavelength may be directed through a second output 208. In various embodiments, the first output and the second output may be collimated (i.e., parallel or the like). In this regard, the first wavelength may provide indicate to a person that the area is being sanitized as the first wavelength would have greater visibility relative to the second wavelength, in accordance with various embodiments. Additionally, in various embodiments, the residual light of the first wavelength through output 207 may be mixed with an additional light source (e.g., light(s) 112 from FIG. 2) to create white light, such as for use as a reading light or the like. Although described with respect to sanitization apparatus 113, any sanitization apparatus disclosed herein (e.g., sanitization apparatuses 123, 133) may be in accordance with sanitization apparatus 113 from FIG. 3, in accordance with various embodiments.

Referring now to FIG. 3, a schematic view of a control system 300 for a portable UVC system 200 is illustrated, in accordance with various embodiments. The control system 300 comprises a controller 302, a memory 304, a light source 306, a trigger 226, and a micro switch 308.

The control system 300 includes the controller 302 and the memory 304 (e.g., a database or any appropriate data structure; hereafter "memory 304" also may be referred to as "database 304"). The controller 302 may include one or more logic devices such as one or more of a central processing unit (CPU), an accelerated processing unit (APU), a digital signal processor (DSP), a field programmable gate array (FPGA), an application specific integrated circuit (ASIC), or the like (e.g., controller 302 may utilize one or more processors of any appropriate type/configuration, may utilize any appropriate processing architecture, or both). In various embodiments, the controller 302 may further include any non-transitory memory known in the art. The memory 304 may store instructions usable by the logic device to perform operations. Any appropriate computer-readable type/configuration may be utilized as the memory 304, any appropriate data storage architecture may be utilized by the memory 304, or both.

The database 304 may be integral to the control system 300 or may be located remote from the control system 300. The controller 302 may communicate with the database 304 via any wired or wireless protocol. In that regard, the controller 302 may access data stored in the database 304. Furthermore, any number of conventional techniques for electronics configuration, signal processing and/or control, data processing and the like may be employed. Also, the processes, functions, and instructions may include software routines in conjunction with processors, etc.

System program instructions and/or controller instructions may be loaded onto a non-transitory, tangible computer-readable medium having instructions stored thereon that, in response to execution by the processor, cause the controller 302 to perform various operations. The term "non-transitory" is to be understood to remove only propagating transitory signals per se from the claim scope and does not relinquish rights to all standard computer-readable media that are not only propagating transitory signals per se. Stated another way, the meaning of the term "non-transitory computer-readable medium" and "non-transitory computer-readable storage medium" should be construed to exclude only those types of transitory computer-readable media which were found in In Re Nuijten to fall outside the scope of patentable subject matter under 35 U.S.C. § 101.

In various embodiments, the controller 302 is in electrical communication with the light source 306 and a micro switch 308. The light source 306 may comprise any light source previously disclosed herein, such as a light emitting diode (LED), a Nd:YAG/LBO laser, a InGaN laser diode, an InGaN laser pump source or the like. The light source 306 may be configured to emit light having a wavelength in accordance with step 102 from method 100 of FIG. 1. For example, the light source 306 may be configured to emit a light with a wavelength between 414 nm and 474 nm, or between 429 nm and 459 nm, or approximately 444 nm.

In various embodiments, the micro switch 308 is in communication with the trigger 226 from FIG. 1. In this regard, in response to the trigger 226 being actuated, the micro switch 308 may close, allowing the micro switch 308 to communicate with the controller 302. In this regard, in response to the micro switch closing, method 100 from FIG. 1 may be performed by the portable UVC system 200 from FIG. 2.

Benefits, other advantages, and solutions to problems have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, solutions to problems, and any elements that may cause any benefit, advantage, or solution to occur or become more pronounced are not to be construed as critical, required, or essential features or elements of the disclosure. The scope of the disclosure is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. Different cross-hatching is used throughout the figures to denote different parts but not necessarily to denote the same or different materials.

Systems, methods, and apparatus are provided herein. In the detailed description herein, references to "one embodiment," "an embodiment," "various embodiments," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

Furthermore, no element, component, or method step in the present disclosure is intended to be dedicated to the public regardless of whether the element, component, or method step is explicitly recited in the claims. As used herein, the terms "comprises," "comprising," or any other variation thereof, are intended to cover a non-exclusive inclusion, such that a process, method, article, or apparatus that comprises a list of elements does not include only those elements but may include other elements not expressly listed or inherent to such process, method, article, or apparatus.

Finally, it should be understood that any of the above described concepts can be used alone or in combination with any or all of the other above described concepts. Although various embodiments have been disclosed and described, one of ordinary skill in this art would recognize that certain modifications would come within the scope of the claims. Accordingly, the description is not intended to be exhaustive or to limit the principles described or illustrated herein to any precise form. Many modifications and variations are possible in light of the above teaching.

## Claims

1. A portable sanitization system (200), comprising:
a portable device (210);
a light source (306) disposed in the portable device (210), the light source configured to emit a light having a wavelength between 414 and 474 nm;
a light converter (230) comprising a nonlinear crystal (234) configured for frequency doubling; and
a light guide (220) extending from the light source to the light converter, the light converter (230) disposed proximal a tip of the light guide (220).

2. The portable sanitization system of claim 1, wherein the light guide (220) comprises a fiber optic cable.

3. The portable sanitization system of claim 1 or 2, wherein the light source (306) comprises a laser pump source.

4. The portable sanitization system of any preceding claim, further comprising a beam directing lens configured to direct converted light from the light converter (230).

5. The portable sanitization system of any preceding claim, wherein the light guide (220) comprises a flexible portion and a rigid portion.

6. The portable sanitization system of claim 5, further comprising a trigger (226) coupled to the rigid portion, and optionally wherein the trigger (226) is in operable communication with the light source, and further optionally wherein the light source (306) is configured to generate the light having the wavelength between 414 and 474 nm in response to the trigger (226) being actuated, and even further optionally wherein the light converter (230) is configured to convert a portion of the light from the wavelength between 414 and 474 nm to a second wavelength between 207 nm and 237 nm.

7. A sanitization device (200), comprising:
a light guide (220);
a light source (306) configured to emit a light having a wavelength between 414 and 474 nm, the light source (306) being portable; and
a light converter (230) having a nonlinear crystal, the light guide (220) extending from the light source (306) to the light converter (230), the light converter (230) configured to convert at least a portion of the wavelength to a second wavelength between 207 nm and 247 nm.

8. The sanitization device of claim 7, further comprising a backpack, the light guide (220) disposed in the backpack.

9. The sanitization device of claim 7 or 8, wherein the light guide (220) comprises a fiber optic cable and a rod, the rod configured to house the light converter (230).

10. The sanitization device of any of claims 7 to 9, further comprising a beam directing lens disposed proximal a tip of the light guide (220), and optionally wherein the light converter (230) is disposed proximal the tip of the light guide (220).

11. The sanitization device of any of claims 7 to 10, wherein the light source (306) comprises one of a light emitting diode (LED), a Nd:YAG/LBO laser, a InGaN laser diode, an InGaN laser pump source.

12. The sanitization device of any of claims 7 to 11, wherein the light guide (220) comprises a flexible portion and a rigid portion, and optionally wherein the flexible portion extends from the light source (306) to the rigid portion, and the rigid portion extends from the flexible portion to a tip of the light guide (220).

13. A method of using a portable sanitization system (200), the method comprising:
generating a light having a first wavelength between 414 and 474 nm;
guiding the light from a first location to a second location;
converting at least a portion of the light from the first wavelength to a second wavelength between 207 and 247 nm;
directing the light having the second wavelength toward a surface; and
in response to directing the light toward the surface, the surface is at least partially sanitized.

14. The method of claim 13, wherein the first location is disposed in a portable device, and wherein the second location is disposed proximal a tip of light guide extending from the portable device.

15. The method of claim 13 or 14, further comprising activating a trigger prior to generating the light having the first wavelength.
